# EUROPÄISCHE PATENTSCHRIFT

(11) **EP 1 549 852 B1**
(45) Veröffentlichungstag und Bekanntmachung des Hinweises auf die Patenterteilung: **21.07.2010**
(21) Anmeldenummer: 03767503.0
(22) Anmeldetag: 04.10.2003
(51) Int. Cl.: F04B 43/08

(54) **PUMPVORRICHTUNG**
PUMP DEVICE
DISPOSITIF POMPE

(30) Priorität: 04.10.2002 DE 10246469
(43) Veröffentlichungstag der Anmeldung: 06.07.2005
(73) Patentinhaber: pfm medical tpm gmbh, 50996 Köln (DE)
(72) Erfinder: GOTTSCHALK, Andreas, 53913 Swisttal-Heimerzheim (DE)
(74) Vertreter: Polypatent
(86) Internationale Anmeldenummer: PCT/EP2003/010998
(87) Internationale Veröffentlichungsnummer: WO 2004/033903

(56) Entgegenhaltungen:
- EP-A- 0 410 872
- US-A- 4 909 710
- US-A- 5 964 583

## Beschreibung

Diese Erfindung betrifft eine Pumpvorrichtung mit einer peristaltischen Antriebseinrichtung zum Pumpen eines Mediums durch eine Leitung mit zumindest einem kompressiblen Abschnitt, enthaltend eine Welle mit versetzt zueinander angeordneten Nocken und aufgefügten Lamellen, wobei eine Zwangszuführung für die Lamellen vorgesehen ist.

Pumpvorrichtungen mit einer peristaltischen Antriebseinrichtung zum Pumpen eines Mediums durch einen Schlauch werden beispielsweise als Transfusionspumpen und Infusionspumpen verwendet. Derartige Geräte sind im Stand der Technik bekannt. Bei den meisten Geräten wird eine Rückstellung der Lamellen durch die Schlauchelastizität erzeugt. Hierbei muss also die Schlauchelastizität so groß gewählt werden, dass ein Zurückschieben der Lamellen durch den Schlauch möglich ist. Üblicherweise werden daher Schläuche aus Silicon verwendet. Ist kein Rückzugsystem vorgesehen, kann es zu einem Klemmen des Schlauches und einer Behinderung des Durchflusses bzw. einem ungewollten Rückfluss kommen.

Aus DE 692 01 966 T2 ist beispielsweise der Aufbau einer Peristaltikpumpe bekannt zum Pumpen eines Fluids von einer Fluidquelle durch eine Leitung, wobei eine Antriebswelle mit Nockenplatten vorgesehen ist, die exzentrisch mit einem Schraubenmuster entlang der Antriebswelle an dieser befestigt und mit dieser verdrehbar sind und mit Fingerplatten, die an der Leitung angreifen und mit den Nockenplatten der Antriebswelle gekoppelt sind. Es ist eine durchgehende Kernwelle als Antriebswelle vorgesehen, auf der die Nockenplatten befestigt sind. Die Fingerplatten weisen jeweils eine Durchgangsöffnung auf, mit der sie auf die Nockenplatten aufgesteckt sind.

Die EP 0 422 855 B1 offenbart eine Peristaltikpumpe mit einer durchgehenden Welle mit daran angeformten Nocken. Auf den Nocken sind Fingerplatten aufgefügt, die jeweils eine im Wesentlichen rechteckige Durchgangsöffnung aufweisen, in die die im Querschnitt runden Nocken eingreifen. Die Fingerplatten bzw. Finger drücken gegen eine Membran, unter der ein Schlauch durchgeführt wird. In dem Patent wird das Vorsehen von vier Fingern bevorzugt. Die einzelnen Finger sind unterschiedlich lang bzw. legen ein Mehrfaches der Strecke zurück, die ein anderer Finger zurücklegt. Es wird zwischen pumpenden und klemmenden Fingern unterschieden, wobei die klemmenden Finger an erster und dritter Position und die pumpenden Finger an zweiter und vierter Position vorgesehen sind. Die Nocken der klemmenden Finger sind nur einseitig und um 180° zueinander verdreht an der Welle vorgesehen, wohingegen die Nocken der pumpenden Finger im Wesentlichen in gleicher Positionierung an der Welle angeordnet sind.

Aus der DE 690 18 208 T2 ist ebenfalls eine Peristaltikpumpe bekannt, bei der eine durchgehende Antriebswelle mit darauf aufgefügten Nocken und mit diesen gekoppelten Fingern vorgesehen ist. Es ist ein einstellbares Ausrichtmittel zur Festlegung einer Drehachse der Nockenwelle vorgesehen, um den Fluidstrom durch einen in die lineare Peristaltikpumpe eingelegten Schlauch zu linearisieren. Durch das einstellbare Ausrichtmittel wird die rotierende Nockenwelle so lange geneigt, bis der Fluidstrom im Wesentlichen linear ist. Die Finger sind im Wesentlichen gleich lang und die Nockenscheiben exzentrisch auf der Antriebswelle montiert.

Aus der DE 693 03 516 T2 ist eine lineare Peristaltikpumpe bekannt, bei der eine motorbetriebene Nockenwelle drehbar in einem Rahmen gehaltert und parallel zu einem flexiblen ebenfalls gehalterten Schlauch angeordnet ist. Die Nockenwelle weist zumindest drei Nocken auf, die in einem Winkel zueinander versetzt angeordnet sind. Für jeden Nocken ist ein Stößel vorgesehen, der zwischen parallel verlaufenden Führungsflächen in einer zur Nockenwelle senkrecht verlaufenden Richtung geführt wird und Endflächen zum Zusammendrücken des flexiblen Schlauchs aufweist. Jeder Nocken ist mit drei Bogenabschnitten versehen, wobei ein erster Abschnitt für die Druckphase, ein zweiter Abschnitt zum Halten des flexiblen Schlauchs in verschlossenem Zustand und ein dritter Abschnitt zur schnellen Freigabe des Schlauches vorgesehen sind.

Auch aus der DE 690 10 194 T2 ist eine Peristaltikpumpe mit einer starren Antriebswelle bekannt. Auch auf dieser Antriebswelle ist eine Vielzahl von Nocken vorgesehen, wobei jeder der Nocken zu dem jeweils benachbarten Nocken winkelversetzt ist. Mit den Nocken zusammenwirkend ist eine Vielzahl von sich hin und her bewegenden Druckfingern vorgesehen, wobei diese von den Nocken angetrieben werden. Die Drehbewegung der Antriebswelle wird in eine lineare Wellenbewegung der Druckfinger umgewandelt. Ein Schlauch ist zwischen den Druckfingern und einer Druckplatte als Widerlager eingeschlossen, wobei die Fluidbewegung durch die Druckfinger bewirkt wird.

Aus der DE 690 08 638 T2 ist eine Transfusionspumpe bekannt, die über einen Antriebsmechanismus Finger bewegt, die aufgrund ihrer Y-Form und einseitigen Befestigung an einem Schenkel über den anderen Schenkel an einem Schlauch angreifen und diesen zusammendrücken können. Der Antriebsmechanismus weist eine Antriebswelle auf, auf der zwölf exzentrische Nockenscheiben aufgefügt sind, die sich an die jeweiligen Fingerfortsätze anlegen. Durch Drehen der Antriebswelle und der darauf befindlichen Nocken werden die Fingerfortsätze ausgelenkt und dadurch ebenfalls die dazu abgewinkelt stehenden Schenkel, die an dem Schlauch anliegen, wodurch dieser fortlaufend zusammengedrückt und eine Fluidförderung bewirkt wird.

In der DE 36 11 643 C2 ist eine peristaltische Schlauchpumpe, die als Einschubeinheit ausgebildet ist, offenbart. Die Schlauchpumpe weist zwölf als Druckglieder wirkende erste und zweite Druckorgane auf, die hintereinander liegend angeordnet sind. Das erste Druckorgan ist dabei als eine rechteckige Platte ausgebildet, die zwei Vorsprünge an der dem zweiten Druckorgan zugewandten Seite aufweist. Das zweite Druckorgan wird durch zwei stabförmige identisch ausgebildete Glieder gebildet. Auf einer Antriebswelle sind in gleichmäßigen Abständen, die etwas länger als die Dicke der Glieder ist, zwölf Nockenscheiben angeordnet, an deren Steuerfläche jeweils eine Platte anliegt. Es ist also wiederum eine durchgehende Antriebswelle mit darauf aufgefügten Nockenscheiben vorgesehen.

In der DE 25 26 060 A1 ist eine peristaltische Schlauchpumpe offenbart, bei der der Schlauch durch eine Anzahl nebeneinander angeordneter identischer Pleuelstangen hindurchgeführt und von diesen getragen wird und sich durch eine Öffnung an einem Ende jeder Pleuelstange, deren anderes Ende an einem Kurbelzapfen einer Kurbelwellenkröpfung drehbar gelagert ist, erstreckt. Die Pleuelstangen werden durch die Kurbelwelle in eine auf- und niedergehende Bewegung versetzt. Die Kurbelwellenkröpfungen sind zueinander in einem Winkel versetzt auf der Kurbelwelle vorgesehen. Da der Schlauch durch die Pleuelstangen hindurchgeschoben ist, folgt er der auf- und niedergehenden Bewegung. Der Schlauch ist mit seinen Enden in einem Pumpenende befestigt und umschließt über seine gesamte Länge eine unbewegliche kreisförmige Stange, die in diesem zentriert ist, sofern keine äußere Beanspruchung durch die Pleuelstangenbewegung auf den Schlauch einwirkt. Bei der fortschreitenden transversalen Wellenbewegung des Schlauches legt sich dieser fortschreitend an der Stange an, wodurch die über und unter der Stange befindlichen Kanäle jeweils geschlossen und geöffnet werden und dadurch die Fluidbewegung in dem Schlauch erzeugt wird. Dies erfolgt ohne Klemmung des Schlauchs, jedoch aufgrund der besonderen Formgebung der Pleuelstangen mit den Durchführöffnungen für den Schlauch und des zusätzlichen Vorsehens der durch den Schlauch hindurchgehenden Stange mit verhältnismäßig großem Aufwand. Neben den vorstehend genannten Peristaltikpumpen mit Antriebswellen und Nocken bzw. Kurbelwelle und Kröpfungen ist es noch bekannt, eine Schlauchpumpe mit gebauter Welle vorzusehen, wie dies insbesondere aus der DE 92 05 733 U1 hervorgeht. Die Schlauchpumpe weist hintereinander angeordnete Schieber auf, die mit ihrer Stirnfläche den Schlauch in rhythmischem Wechsel beaufschlagen. Die Schieber werden von winkelversetzt angeordneten Exzenterscheiben gesteuert, die sich um eine gemeinsame Achse drehen. Die gemeinsame Achse wird von Achsstummeln gebildet, die jeweils auf der einen Seite einer jeweiligen Exzenterscheibe vorstehen und in korrespondierende Vertiefungen der jeweils benachbart liegenden Exzenterscheibe eingefügt werden. Zur weiteren Sicherung der Achsstummel aneinander sind außerdem Steckzapfen vorgesehen, die jeweils in Löcher benachbarter Exzenterscheiben eingefügt werden. Durch den jeweiligen Versatz von Löchern und Steckzapfen wird der Versatz der Exzenterscheiben zueinander gebildet. Diese Form des Aufbaus einer mit Exzenterscheiben versehenen Welle oder Achse erweist sich als recht aufwendig aufgrund der großen Anzahl der Einzelteile, die zueinander passend hergestellt werden müssen.

Aus der DE 298 05 173 U1 ist eine lineare Membranpumpe bekannt, bei der ein Förderkanal vorgesehen ist, wobei die Welle schneckenförmig ist.

Die US 4,909,710, welche als nächstkommender Stand der Technik angesehen wird, offenbart eine Pumpvorrichtung mit einer einteiligen und mit zueinander versetzten Nockenscheiben auf ihrem Umfang versehenen Welle. Die Nockenscheiben sind dabei einteilig mit der Welle. Hierdurch wird wie bei den auf eine Welle aufgefügten Nockenscheiben ebenfalls keine große Durchflussrate bei kleiner Bauart der Pumpvorrichtung ermöglicht. Es liegt dieselbe Beschränkung vor wie beispielsweise im Stand der Technik der DE 692 01 966 T2.

Aus der DE 32 02 251 C2 ist eine Infusionsschlauchpumpe bekannt, die ein Pumpglied mit mehreren quer zur Pumpstrecke wellenförmig fortschreitend und synchron bewegbare Peristaltikschieber aufweist. Diese sind an ihrem einen Ende auf Kurbelzapfen drehbar gelagert. Die Kurbelzapfen sind exzentrisch auf einer Pumpenachse gelagert, so dass sie im Betrieb die Peristaltikschieber quer zur Pumpstrecke hin und her verschieben.

Der vorliegenden Erfindung liegt nun die Aufgabe zugrunde, eine weniger aufwendige Pumpvorrichtung mit peristaltischer Antriebseinrichtung zu bilden, die eine möglichst gute Durchflussrate bei kleiner Bauart zur Verfügung stellt, und bei der ein Klemmen eines Schlauchs bzw. einer Leitung im Bereich ihres zumindest einen kompressiblen Abschnitts vermieden werden kann.

Die Aufgabe wird durch eine Pumpvorrichtung nach dem Oberbegriff des Anspruchs 1 dadurch gelöst, dass Nocken Nockensegmente sind und die Welle kemwellenlos ohne einen durchgehenden Kernbereich oder zur Stabilitätserhöhung mit einem dünnen durchgehenden Kernbereich mit einem Durchmesser von unter 3 mm mit zueinander versetzten, aneinander angrenzenden Nockensegmenten ausgebildet ist. Bei einer Welle für eine Pumpvorrichtung mit einer peristaltischen Antriebseinrichtung, wobei die Welle einstückig geformt ist, wird die Aufgabe dadurch gelöst, dass die Welle kemwellenlos ohne einen durchgehenden Kernbereich oder zur Stabilitätserhöhung mit einem dünnen durchgehenden Kernbereich mit einem Durchmesser von unter 3 mm mit zueinander versetzten, aneinander angrenzenden Nockensegmenten ausgebildet ist. Weiterbildungen der Erfindung sind in den abhängigen Ansprüchen definiert.

Dadurch wird eine Pumpvorrichtung mit einer peristaltischen Antriebseinrichtung vorgesehen, die sehr klein ausgebildet werden kann bei einer hohen Durchflussrate, die insbesondere bei 8 l/h liegt. Dies ist mit kleinen Pumpenmodellen des Standes der Technik nicht möglich. Bei Vorsehen einer Antriebswelle mit darauf aufgefügten Nockenscheiben, wie sie vorstehend im Stand der Technik beschrieben sind, sind die für derart hohe Durchflussraten erforderlichen Hübe nicht zu erzielen. Mit den Pumpvorrichtungen nach dem

Stand der Technik, bei denen bei der Vor- und Zurückbewegung geführte Wellen vorgesehen sind, sind bestenfalls Verhältnisse zwischen Lamellenhöhe und -hub von 7,5:1 und schlechter möglich. Die bekannten Lamellen weisen eine Höhe von beispielsweise 30 mm bei einer Öffnungsweite der Öffnungen zum Durchführen von Nockenscheiben von 23 mm auf. Mit den Konzepten der durchgehenden Antriebswellen mit aufgefügten Nockenscheiben nach dem Stand der Technik sind hierbei nur Hübe von 4 mm möglich. Aufgrund des Vorsehens einer Zwangsführung für die Lamellen bei der Vor- und Zurückbewegung wird von diesen stets ein definierter Zustand eingenommen und kann das Risiko des Klemmens des Schlauchs, was zu dessen Verletzung führen kann, vermieden werden. Die Lamellen werden nach dem Absenken auf den Schlauch stets wieder von der Welle zurückgezogen. Dieser Vorteil einer Zwangsführung ist vorteilhafterweise in Verbindung mit einem besonders hohen Hub der Lamellen möglich. Mit den Pumpvorrichtungen des Standes der Technik ist dies nicht möglich. In vielen Fällen bewirkt dort die Elastizität des Schlauches eine Rückstellung der Lamellen. Da das Risiko eines Abklemmens eines Schlauches trotz der hohen Hübe bei der vorliegenden Erfindung nicht zu befürchten steht, können auch billigere Schläuche als die üblichen Siliconschläuche verwendet werden, beispielsweise PVC-Schläuche. Hierdurch werden vorteilhaft Betriebskosten eingespart.

Die Welle ist kemwellenlos. Es wird also keine durchgehende Welle vorgesehen, auf die Nockenscheiben aufgefügt sind oder in die Nockenscheiben integriert sind, wie bei der US 4,909,710. Es wird auch keine gebaute Welle verwendet, wie dies in der DE 92 05 733 U1 offenbart ist. Vielmehr wird eine aus aneinandergesetzten Nockensegmenten ohne zentrale Kernwelle gebildete Welle verwendet. Diese ist jedoch einstückig, besonders bevorzugt im Gussverfahren hergestellt. Hierdurch wird es möglich, die Nockensegmente mit solchen Abstufungen zueinander zu versehen, dass auch bei besonders kleinen Lamellenabmessungen sehr große Hübe erreicht werden können. Die Lamellen sind hierbei auf die Welle aufgefügt. Durch das Einsparen einer durchgehenden Kernwelle und das bloße Aneinanderfügen von Nockensegmenten können beliebige Formen der Welle erzielt werden, die vorzugsweise an den jeweiligen Anwendungsfall anpassbar sind. Welle und Lamellen bestehen bevorzugt aus einem Kunststoffmaterial. Die Wahl des Kunststoffmaterials kann sich bevorzugt nach den Festigkeitsanforderungen richten. Auch Faserverbundstoffe und Kombinationen aus Metallen, Kunststoffen, Faserstoffen etc. sind möglich.

Bevorzugt weist die Welle entweder keinen durchgehenden Kernbereich oder einen dünnen durchgehenden Kernbereich auf, insbesondere einen durchgehenden Kernbereich mit einem Durchmesser von unter 3 mm, insbesondere 2 mm oder weniger oder 1 mm oder weniger. Hierdurch kann die Stabilität der Welle vergrößert, insbesondere verdoppelt werden. Es wird also keine durchgehende Kernwelle vorgesehen, auf die die Nockenscheiben aufgefügt sind, sondern ein durchgehender Kernbereich, den jedes Nockensegment der Welle bzw. jedes Segment der Welle durchläuft. Die Knickstabilität wird dadurch ersichtlich vergrößert gegenüber einer Ausführungsform ohne durchgehenden Kernbereich, da ein zwar schmaler, jedoch durchgehender stabiler Bereich geschaffen wird. Bei einer Ausführungsform im Wesentlichen ohne durchgehenden Kernbereich kann ein noch besseres, also geringeres, Verhältnis von Lamellenhöhe zu Hub erzeugt werden, wobei jedoch die Welle aufgrund ihrer freien Formgestaltung weniger knickstabil wird. Das Verhältnis von Nockensegmentdurchmesser zum Durchmesser eines durchgehenden Kernbereichs beträgt vorzugsweise weniger als 4:1, insbesondere weniger als 3:1, insbesondere weniger als 2:1, insbesondere weniger als 1:1. Bei einem Nockensegmentdurchmesser von 6 mm und einem Kernbereichsdurchmesser von 2 mm ergibt sich beispielsweise ein Verhältnis von 3:1. Die Belastbarkeit einer solchen Welle wird durch die Größe der Kontaktflächen zwischen den einzelnen Nockensegmenten bestimmt. Insbesondere kann bei Weglassen eines durchgehenden Kernbereichs eine Stabilitätserhöhung durch Erhöhen der Anzahl der Nockensegmente erzielt werden, da sich hierdurch die Kontakt- oder Konnektionsfläche zwischen den einzelnen Nockensegmenten vergrößern lässt aufgrund eines kleineren Versatzes zwischen den Nockensegmenten.

Vorzugsweise ist eine ungerade oder gerade Anzahl von Nockensegmenten vorgesehen. Vorzugsweise sind die Nockensegmente so zueinander versetzt, dass nur ein Nockensegment einen maximalen Abstand zu einer fiktiven Mittellinie der Welle aufweist. Hierdurch wird es ermöglicht, dass durch die Lamellen ein in die Pumpvorrichtung eingelegter Schlauch einerseits nicht vollständig abgeklemmt, andererseits jedoch ohne dass ein Rückfluss befürchtet werden müsste, fortschreitend einseitig so zusammengedrückt wird, dass eine Fluidströmung in dem Schlauch erzeugt werden kann.

Bevorzugt ist die Anordnung der Nockensegmente entlang der Welle so gewählt, dass ein Pumpvorgang in zwei Richtungen (erste und entgegengesetzte zweite Richtung) möglich ist.

Besonders bevorzugt ist ein gleichmäßiger Versatz der Nockensegmente vorgesehen, insbesondere ein Versatz von 40° bei neun Nockensegmenten. Je nach Anwendungsfall kann jedoch auch ein ungleichmäßiger Versatz der Nockensegmente vorgesehen werden, da diese aufgrund des Vorsehens einer kernwellenlosen Welle beliebig zueinander versetzt ausgebildet werden können. Ein gleichmäßiger Versatz der Nockensegmente trägt jedoch zu einem symmetrischeren Aufbau und gleichmäßigeren Pumpvorgang bei.

Vorzugsweise wird die Pumpvorrichtung als Infusionspumpe, Transfusionspumpe, für die Dialyse oder als Schlauchpumpe für einen anderen medizinischen Zweck verwendet. Gerade bei dem Anwendungsfall in der Dialyse kann eine Zerstörung des Blutplasmas verhindert werden, was ansonsten durch Klemmen des Schlauchs auftreten könnte. Wichtig ist hierbei lediglich, dass ein Volumen vorne und hinten, also beim ersten und beim letzten Nockensegment, dicht eingeschlossen werden kann und die übrigen Lamellen bzw. Nockensegmente zur Volumenverkleinerung dienen. Die erste und letzte Lamelle werden bevorzugt als Ventil geschaltet und die übrigen Lamellen werden bevorzugt so eingestellt, dass in jeder Position zumindest ein schmaler Spalt zwischen den Wänden des durch die Lamellen beaufschlagten Schlauches verbleibt. Außerdem ist vorteilhaft eine Nutzung der Pumpvorrichtung für ein Zweikanalsystem möglich, indem beidseitig an die Welle Lamellen angefügt und zwei Schläuche an der Welle beidseitig vorbeigeführt werden.

Vorzugsweise besteht die Welle aus einem Kohlefaserwerkstoff, einem glasfaserverstärkten Polymer oder einem anderen stabilen und maßhaltigen Material. Gerade bei der Verwendung eines Kohlefaserwerkstoffs sind große Belastungen möglich, insbesondere Belastungen bis 20 kg. Ein Brechen der Welle steht hierbei auch bei besonders ausgefallener Gestaltung der Welle ohne Kernwellenbereich nicht zu befürchten. Die Verwendung eines glasfaserverstärkten Polymers erweist sich ebenfalls als vorteilhaft, insbesondere im Lagerbereich der Welle, da eine Schmierung des Lagers nicht möglich ist und dieses bei Verwendung eines solchen glasfaserverstärkten Polymers keiner Schmierung bedarf. Auch kann mit den vorstehend genannten Werkstoffen bzw. alternativen stabilen und maßhaltigen Werkstoffen eine Genauigkeit vom ersten bis zum letzten Nockensegment von insbesondere 5/100 mm erzielt werden. Zur Lagerung der Welle können bevorzugt Gleitlager oder Kugellager verwendet werden.

Vorteilhaft entspricht bei den zur Verwendung mit einer solchen Pumpvorrichtung vorgesehenen Lamellen die Durchgangsöffnung in Längsrichtung im Wesentlichen dem Außendurchmesser von Nockensegmenten der Welle, auf die die Lamellen auffügbar oder aufgefügt sind. Hierdurch wird sichergestellt, dass die Lamellen von der Welle bzw. deren Nockensegmenten sicher mitgenommen werden, ohne auf diesen zu klemmen oder zu verrutschen. Besonders bevorzugt ist die Durchgangsöffnung im Wesentlichen ein Langloch mit einer größeren Erstreckung quer zur Längsrichtung der Lamelle. Die Abmessung in Längsrichtung des Langlochs entspricht vorzugsweise im Wesentlichen dem von den rotierenden Nockensegmenten umschriebenen Außendurchmesser. Hierdurch ist eine freie Beweglichkeit der Lamelle auf dem Nockensegment in der Richtung quer zur Längsrichtung der Lamelle möglich, was ein Verklemmen der Lamelle bei der Bewegung der Welle verhindert, zugleich aber deren Mitnahme durch die Welle sicherstellt. Die Lamelle bleibt somit nicht ungewollt auf dem Schlauch klemmend stehen, sondern wird, ohne dessen Schlauchelastizität zu benötigen, fortschreitend von diesem wieder weggezogen.

Vorzugsweise ist eine Gegendruckplatte zum Anlegen der Leitung, insbesondere eines Schlauchs und Gegenhalten des von den Lamellen auf die Leitung bzw. den Schlauch ausgeübten Drucks vorgesehen. Besonders bevorzugt ist die Gegendruckplatte durch eine oder mehrere Federn innerhalb des Gehäuses der Pumpvorrichtung abgefedert, insbesondere durch Tonnenfedern, Blattfedern oder eine andere Federart. Hierdurch ist ein individuelles Einstellen des Gegendrucks auf die Leitung möglich, da bei Ändern der Anzahl und Art der Federn in Kombination mit der Gegendruckplatte jeweils unterschiedliche Gegenkräfte erzeugt werden können. Alternativ kann die Gegendruckplatte ohne Federn als festes Element vorgesehen werden. Der Federweg kann dann durch die Elastizität der Leitung bzw. des Schlauchs mit ausreichend großer Wandstärke aufgebracht werden.

Zur näheren Erläuterung der Erfindung werden im Folgenden Ausführungsbeispiele anhand der Zeichnungen näher beschrieben. Diese zeigen in:
- Figur 1: eine perspektivische Ansicht einer ersten Ausführungsform einer erfindungsgemäßen Pumpvorrichtung, die zur Verdeut- lichung teilweise aufgeschnitten dargestellt ist,
- Figur 2: eine Querschnittsansicht der Ausführungsform der Pumpvor- richtung gemäß Figur 1,
- Figur 3: eine perspektivische Ansicht einer zweiten Ausführungsform einer erfindungsgemäßen Pumpvorrichtung mit dem Detail der Welle, Lamellen, eines Schlauchs und einer Gegendruckplatte,
- Figur 4: eine Draufsicht auf die Ausführungsform gemäß Figur 3,
- Figur 5: eine Schnittansicht durch die Ausführungsform gemäß Figur 3 entlang der Schnittlinie A-A in Figur 4,
- Figur 6: eine perspektivische Ansicht einer weiteren Ausführungsform der erfindungsgemäßen Pumpvorrichtung mit dem Detail der Welle, Lamellen, eines Schlauchs und einer Gegendruckplatte,
- Figur 7: eine Draufsicht auf die Ausführungsform gemäß Figur 6,
- Figur 8: eine Querschnittsansicht der Ausführungsform gemäß Figur 6 entlang der Schnittlinie B-B gemäß Figur 7,
- Figur 9: eine Darstellung der fortschreitenden Bewegung von Lamellen und Welle während eines Pumpvorgangs in der Abfolge von zwölf Schritten,
- Figur 10: eine perspektivische Ansicht einer weiteren Ausführungsform des Details von Schlauch, Welle, Lamellen und Gegendruckplatte einer erfindungsgemäßen Pumpvorrichtung,
- Figur 11: eine perspektivische Schnittansicht der Ausführungsform gemäß Figur 10,

- Figur 12: eine Querschnittansicht der Ausführungsform gemäß Figur 10 durch eine Lamelle, und
- Figur 13 bis 16: Prinzipskizzen von verschiedenen Ausführungsformen von Wellen mit unterschiedlichen Nockensegmentdurchmessern und gleichem Hub mit und ohne durchgehendem Kernbereich.

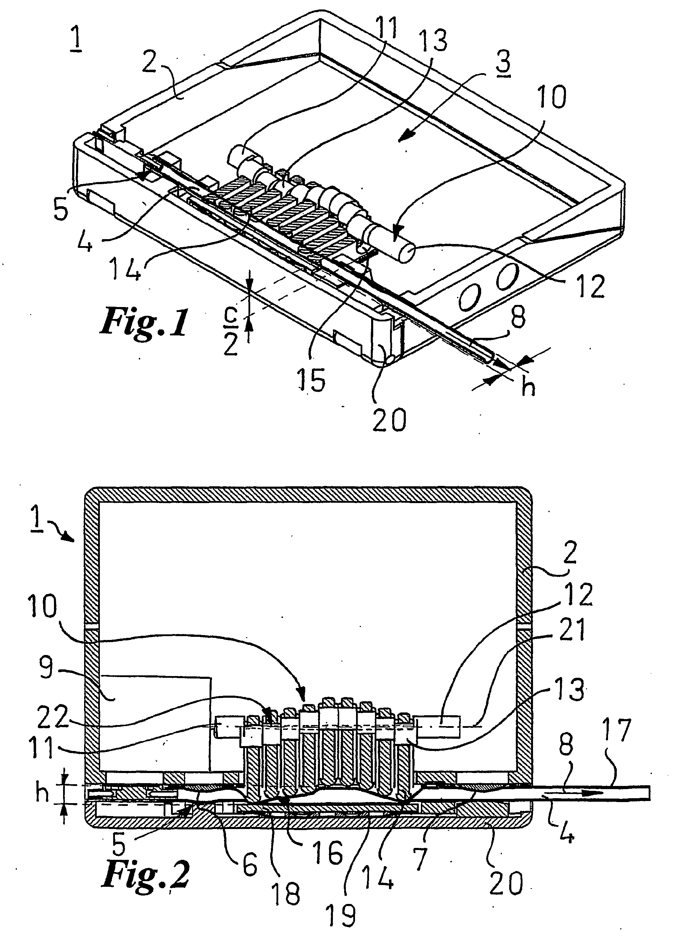

Figur 1 zeigt eine perspektivische Ansicht einer ersten Ausführungsform einer erfindungsgemäßen Pumpvorrichtung 1, die aufgeschnitten dargestellt ist. Die Pumpvorrichtung weist ein Gehäuse 2 auf, in dem eine peristaltische Antriebseinrichtung zum Erzeugen eines Fluidstroms in einem Schlauch 4 angeordnet ist. Der Schlauch besteht bevorzugt aus einem elastischen Material bzw. weist zumindest einen Abschnitt auf, der aus einem kompressiblen Material besteht. Mit diesem Abschnitt wird der Schlauch in einen Einlegebereich 5 eingelegt, wie dies auch der Querschnittsansicht in Figur 2 entnommen werden kann. Vor und hinter dem Einflussbereich der peristaltischen Antriebseinrichtung sind Druck- und Durchflusssensoren 6,7 angeordnet. Die Durchflussrichtung des Mediums innerhalb des Schlauches durch diesen ist durch einen Pfeil 8 angegeben.

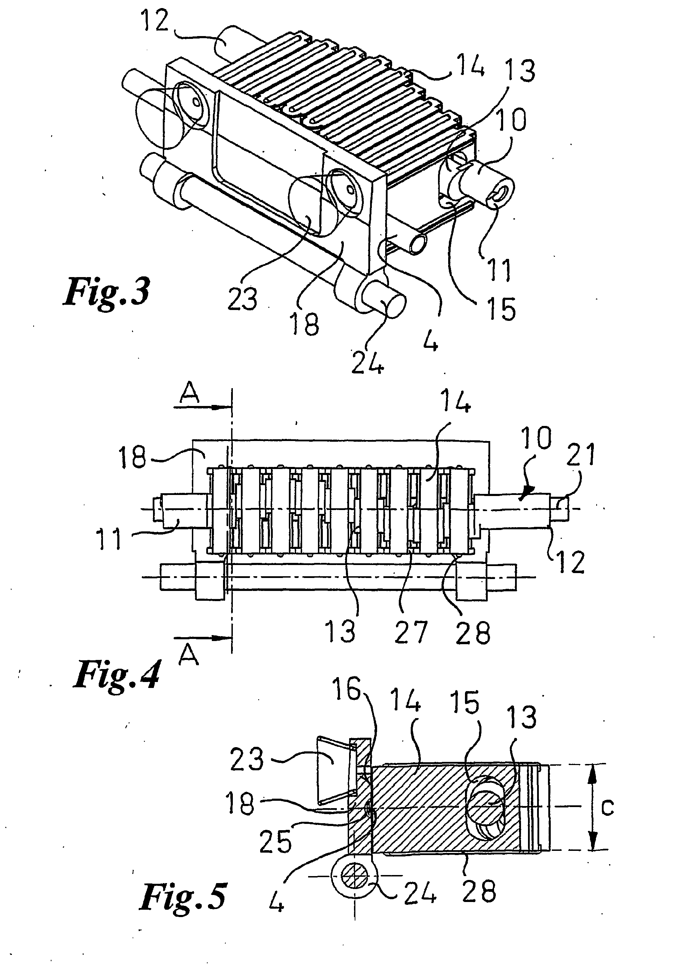

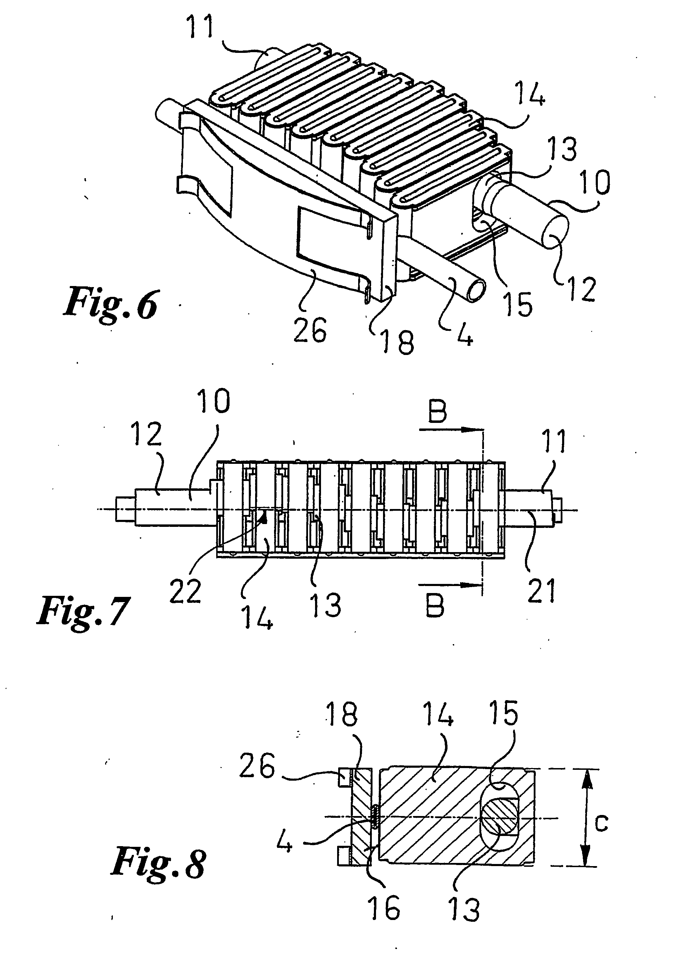

Die peristaltische Antriebseinrichtung 3 weist einen Antriebsmotor 9 auf, der an einer Welle 10 an deren einem Ende 11 angreift. Ein zweites Ende 12 der Welle ist innerhalb des Gehäuses gelagert, was jedoch den Figuren nicht im Detail entnommen werden kann. Die Welle 10 weist versetzt aneinandergefügte Nockensegmente 13 auf. Auf die Nockensegmente sind Lamellen 14 aufgefügt. Zu diesem Zweck weisen diese jeweils eine Durchgangsöffnung 15 auf. Dies kann insbesondere besser den Figuren 5 und 8 entnommen werden. Die Lamellen greifen mit ihren Stirnflächen 16 an der Außenseite 17 des Schlauchs an.

Auf der der peristaltischen Antriebseinrichtung hinsichtlich des Schlauchs gegenüber liegenden Seite ist eine Gegendruckplatte 18 angeordnet, gegen die die Stirnflächen der Lamellen arbeiten. Die Gegendruckplatte ist über eine Feder 19 federnd in einer Klappe 20, die zum Einlegen des Schlauchs aufgeklappt werden kann, gelagert. Während der Drehbewegung der Welle werden die Lamellen abwechselnd aufgrund ihres Versatzes zueinander gegen den Schlauch bewegt und drücken diesen gegen die Gegendruckplatte 18. Um ein Abklemmen bzw. zu starkes Klemmen des Schlauches zu vermeiden, was unter anderem auch zu einem Zerquetschen von Blutplasma führen kann, ist die federnde Lagerung der Gegendruckplatte in Verbindung mit der besonderen Ausgestaltung der Welle 10 vorgesehen.

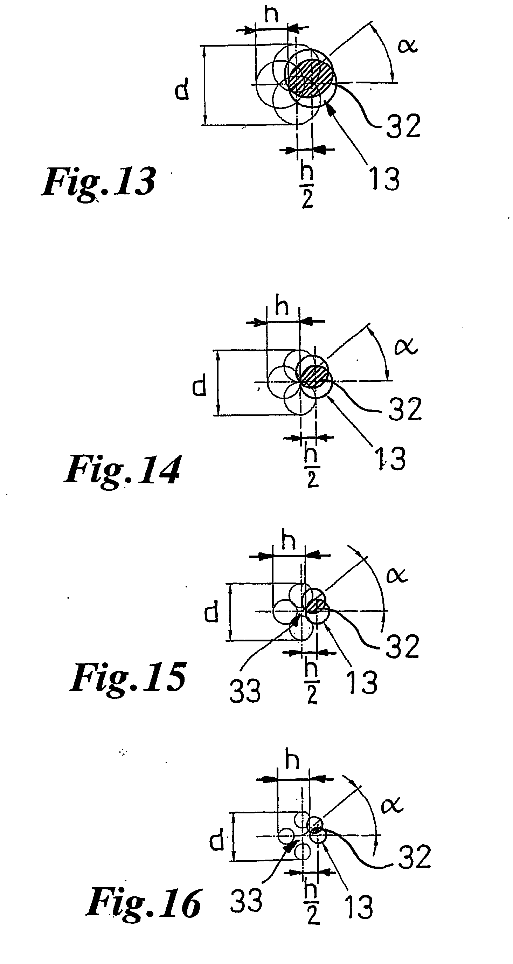

Die in den Figuren 1 und 2 dargestellte Welle ist kernwellenlos, d. h. es ist keine durchgehende Kemwelle vorgesehen, auf die Nockenscheiben aufgefügt sind, sondern eine aus Nockensegmenten einstückig aufgebaute Welle. Aufgrund des Weglassens einer Kernwelle kann ein beliebiger Versatz zwischen den einzelnen Nockensegmenten vorgesehen werden. Insbesondere können die Nockensegmente so weit außerhalb einer gedachten Mittellinie 21 der Welle 10 vorgesehen werden, dass bei dem Vorsehen einer Kemwelle, wie dies im Stand der Technik offenbart ist, ein solches Nockensegment gar nicht mehr auf die Welle aufgebracht werden könnte (siehe Figuren 15 und 16). Hierdurch ist eine Vergrößerung des Hubes h gegenüber dem Stand der Technik möglich. Zugleich können auch die Lamellen geringere Abmessungen aufweisen, und insbesondere eine geringere Höhe c. Das Verhältnis zwischen der Lamellenhöhe und deren Hub beträgt etwa 4:1 oder besser, insbesondere etwa 3,5:1, etwa 3:1, etwa 2,5:1, etwa 2:1, etwa 1,5:1, etwa 1:1 oder weniger. Auch Verhältnisse, die zwischen den angegebenen liegen, sind möglich. Im Stand der Technik sind lediglich Verhältnisse zwischen Lamellenhöhe und Hub bei geführten Wellen, bei denen die Lamellen von. der Welle weg von dem zu komprimierenden Schlauch zurückgezogen werden, von 7,5:1 oder 8:1 und schlechter möglich.

Beispielsweise beträgt der Hub h 6 mm und die, Lamellenhöhe c 13 mm, wodurch sich ein Verhältnis von 2,17:1 ergibt. Im Stand der Technik sind Lamellen mit Höhen von 30 mm bei einem Hub von 4 mm üblich, wodurch sich ein Verhältnis von 7,5:1 ergibt. Mit der Pumpvorrichtung 1 gemäß der vorliegenden Erfindung ist somit ein höherer Durchsatz an Fluidvolumen durch den Schlauch möglich, das insbesondere bei 8 l/h liegen kann. Bei Pumpvorrichtungen mit so kleinen Abmessungen ist dies im Stand der Technik nicht möglich. Um diese Durchsätze zu erzielen, wären mit den Bauformen des Standes der Technik sehr große Abmessungen erforderlich. Diese sollen jedoch gerade bei medizinischen Anwendungen wie Infusions- und Transfusionspumpen sowie in der Dialyse möglichst vermieden werden, da die Patienten vorteilhaft die Pumpvorrichtungen bei sich tragen. Eine kleine Bauform ist mit der Pumpvorrichtung 1 gemäß der Erfindung möglich. Die Länge der Lamellen kann, anders als in den Figuren dargestellt, sehr gering sein. Eine bessere Stabilität der Führung der Vor- und Rückbewegung der Lamellen kann jedoch durch einen längeren Lamellenkörper erzielt werden.

Um eine größere Stabilität der Welle 10 zu erhalten, kann ein Kernbereich 22 durchgehend sein, d. h. dieser wird von allen Nockensegmenten durchlaufen. Gemäß Figur 2 ist der Kernbereich 22 sehr klein. Beispielsweise kann der Kernbereich 0,8 mm betragen, wodurch sich der Lamellenhub bei gleicher Bauform von 6 mm auf 5,2 mm verringert. Hierbei wäre jedoch immer noch ein Verhältnis zwischen Lamellenhöhe und -hub von 2,5:1 gegeben. Dies ist somit immer noch gravierend besser als im Stand der Technik bei verbesserter Stabilität der Welle 10.

Wie Figur 1 und 2 entnommen werden kann, sind die Nockensegmente 13 zueinander im Wesentlichen gleichmäßig versetzt. Bei den in diesen Figuren dargestellten neun Nockensegmenten ist ein Versatz von 40° zwischen den Nockensegmenten gewählt, so dass sich bei einer Umdrehung der Welle jede Lamelle einmal mit ihrer Stirnfläche an der Schlauchaußenseite anlegt. Selbstverständlich kann auch ein anderer Versatz zwischen den Nockensegmenten gewählt werden sowie eine beliebig andere Anzahl von Nockensegmenten.

In den Figuren 3 bis 5 ist eine weitere Ausführungsform eines Teils der Pumpvorrichtung dargestellt. Hierbei sind anstelle einzelner Federn, wie in der Ausführungsform nach Figur 2, zwei Tonnenfedern 23 auf der Gegendruckplatte 18 vorgesehen, wie besonders gut den Figuren 3 und 5 zu entnehmen ist. Die Gegendruckplatte ist über ein Scharnier 24 von den Lamellen weg schwenkbar angeordnet. Der Schlauch liegt in einer Mulde 25 in der Gegendruckplatte, wie dies in Figur 5 gezeigt ist. In der Darstellung gemäß Figur 5, die eine Schnittansicht entlang der Linie A-A gemäß Figur 4 ist, drückt die Lamelle 14 mit ihrer Stirnfläche 16 gegen die Gegendruckplatte 18. Durch das Vorsehen der Mulde 25 kann der Schlauch an dieser Stelle nicht vollständig zusammengedrückt und abgeklemmt werden. Jedoch wird vorzugsweise der Abstand der Welle bzw. der Nockensegmente in der äußersten Position zu der Gegendruckplatte so eingestellt, dass ein Abklemmen des Schlauchs ohnehin nicht zu befürchten steht. Seitliche Stege 27 in Längsrichtung der Lamellen auf deren Seitenflächen, wie besonders in Figur 4 und 5 zu sehen, dienen der Verringerung der Kontaktfläche zwischen den Lamellen und damit der Verminderung von Reibungsverlusten. Seitliche Stege 28 auf den Stirnflächen der Lamellen dienen der Reibungsverminderung.

Wie insbesondere der Figur 4 zu entnehmen ist, ist die in dieser Ausführungsform gezeigte Welle 10 kernwellenlos und ohne Kernbereich 22 ausgebildet. Das von links gerechnet achte Nockensegment ist in seiner ausgefahrenen Position so weit von der Mittellinie 21 entfernt bzw. liegt mit seiner Außenfläche an dieser so an, dass der Kernbereich zu Null wird. Die Außenfläche des Nockensegments kann sogar außerhalb der Mittellinie, in Figur 4 unterhalb der Mittellinie 21, liegen, was zu einem noch größeren Hub führt. Die Grenze wird hierbei lediglich durch die Stabilität der Welle gegeben, die auch hohe Belastungen durch große Durchflussvolumina vorteilhaft aushalten sollte.

Entsprechend den Figuren 3 und 5 ist die Durchgangsöffnung 15 durch die Lamellen 14 als Langloch ausgebildet. In Längsrichtung der Lamellen entspricht hierbei die Öffnungsweite der Durchgangsöffnung im Wesentlichen dem Außendurchmesser des Nockensegments. In der hierzu quer angeordneten Richtung, also in Richtung der Höhe der Lamelle, öffnet sich die Durchgangsöffnung recht weit, so dass eine ausreichende Beweglichkeit während der Wellenumdrehung gegeben ist. Die Öffnungsweite in dieser Richtung wird durch den Abstand der Außenflächen der Nockensegmente zur Mittellinie 21 bestimmt. Damit sich die Lamellen lediglich linear zu dem Schlauch hin und von diesem weg bewegen, nicht jedoch in der Richtung senkrecht hierzu, ist die Öffnungsweite des Langloches ausreichend groß gewählt. Ein Klemmen der Lamelle auf dem Nockensegment wird vorzugsweise durch die Abmessungen und eine geeignete Materialwahl von Lamelle und Nockensegmenten bzw. Welle vermieden. Beispielsweise wird die Welle aus einem Kunststoff, insbesondere aus Kohlefaserwerkstoff, insbesondere CFK, hergestellt. Es kann jedoch auch ein glasfaserverstärktes Polymer gewählt werden, was sich besonders vorteilhaft hinsichtlich der Lagerung der Welle im Gehäuse und an der Antriebseinheit, insbesondere dem Motor 9, bewährt. Außerdem ist mit diesen Werkstoffen eine hohe Genauigkeit zwischen dem ersten und letzten Nockensegment möglich, wodurch ein Klemmen in den im Wesentlichen gleichen Durchgangsöffnungen der Lamellen vermieden werden kann. Hierbei werden beispielsweise Genauigkeiten von 5/100 mm erzielt. Die Lamellen bestehen beispielsweise ebenso aus einem Kunststoff. Die Materialwahl für Lamellen und Welle wird bevorzugt von den auftretenden Beanspruchungen abhängig gemacht, insbesondere ein Kunststoff in Abhängigkeit von der erforderlichen Festigkeit gewählt.

Eine weitere alternative Ausführungsform der Antriebseinrichtung der erfindungsgemäßen Pumpvorrichtung ist in den Figuren 6 bis 8 gezeigt. Im Unterschied zu der Ausführungsform gemäß Figuren 3 bis 5 ist eine Blattfeder 26 anstelle der Tonnenfedern 23 zum federnden Abstützen der Gegendruckplatte 18 vorgesehen. Bei Vorsehen einer Blattfeder ist es nicht unbedingt erforderlich, die Gegendruckplatte als Klappscharnier auszubilden, sondern es kann vielmehr die Gegendruckplatte nach dem Einlegen des Schlauchs vor diesen gefügt und die Blattfeder nachfolgend eingeschoben werden. Die Gegendruckplatte 18 gemäß dieser Ausführungsform weist keine Mulde zum Einfügen des Schlauchs bzw. eines Teils von diesem auf, wie dies insbesondere in Figur 8 zu sehen ist. Anstelle der dargestellten Ausführungsform können beispielsweise ein, zwei oder mehrere dünne Blattfedern an den Seiten der Gegendruckplatte vorgesehen werden.

Figur 8 zeigt einen Schnitt durch die von der Antriebsseite her gesehen erste Lamelle, wobei die Figuren 6 und 7 im Vergleich zu den Figuren 3 und 4 eine um 180° gewendete Antriebseinheit zeigen. Figur 7 kann dabei im Bereich des achten Nockensegments entnommen werden, dass ein ganz geringer Kernbereich 22 bei dieser Welle 10 vorgesehen ist. Der Kernbereich ist nur in diesem Bereich angedeutet, um die Klarheit der übrigen Darstellung nicht herabzusetzen.

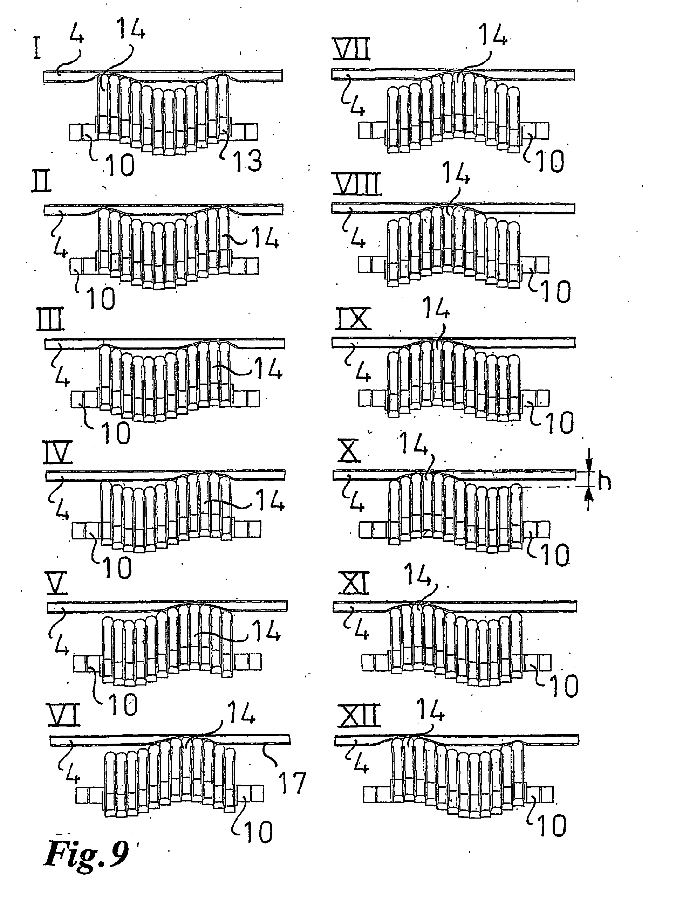

In Figur 9 ist die Abfolge der fortschreitenden Bewegung der Lamellen bei einer Wellenumdrehung in 12 Schritten I bis XII dargestellt. Im Schritt I klemmt die von links gesehen erste, von rechts gesehen letzte Lamelle, nämlich die zwölfte, den Schlauch 4. Die von rechts gesehen erste Lamelle ist kurz davor, den Schlauch zu klemmen. Im Schritt II liegt der umgekehrte Fall vor, die von rechts gesehen letzte Lamelle wurde ein Stück zurückgezogen, wohingegen die von rechts gesehen erste Lamelle nun auf den Schlauch drückt. Hierdurch kann ein Fluidvolumen sicher zwischen der ersten und letzten Lamelle eingeklemmt werden, was insbesondere bei der Dialyse sehr wichtig ist, um den Rückfluss des Fluids bzw. Mediums zu dem Patienten sicher zu verhindern.

In dem Schritt III sind erste und letzte Lamelle wieder ein Stück zurück gewichen und die von rechts gesehen zweite Lamelle klemmt nun den Schlauch. Bei Fortschreiten der Bewegung klemmt im Schritt IV die von rechts gesehen dritte Lamelle den Schlauch und im Schritt V die von rechts gesehen vierte Lamelle. Die übrigen Lamellen weichen jeweils fortschreitend ein Stück zurück. Bei weiterer Drehung der Welle 10 klemmt im sechsten Schritt die von rechts gesehen fünfte Lamelle den Schlauch und im siebten Schritt die von rechts gesehen sechste Lamelle. Im sechsten Schritt ist die letzte Lamelle maximal von der Schlauchaußenseite 17 weggezogen, die von rechts gesehen erste Lamelle fast maximal. Zwischen der am weitesten von der Schlauchoberfläche weggezogenen und der gerade klemmenden Lamelle kann der Hub h bestimmt werden, also das Maß, um das die Lamellen maximal hin und her bewegt werden.

Im Schritt VIII klemmt die von rechts gesehen siebte Lamelle den Schlauch und im Schritt IX die von rechts gesehen achte Lamelle. Im Schritt VIII nähert sich die von rechts gesehen letzte Lamelle wieder dem Schlauch, wohingegen die von rechts gesehen erste Lamelle nun maximal von seiner Außenseite entfernt ist. Hingegen nähert sich im Schritt IX auch die erste Lamelle wieder der Schlauchaußenseite. Im Schritt X klemmt die von rechts gesehen neunte Lamelle und im Schritt XI die von rechts gesehen zehnte Lamelle. In diesem Schritt tritt auch die von rechts gesehen letzte Lamelle wieder in den Klemmvorgang ein und vermindert das Durchgangsvolumen des Schlauchs bereits um ein Stück. Im Schritt XII klemmt die von rechts gesehen elfte Lamelle und die von rechts gesehen erste Lamelle beginnt wieder, das Schlauchvolumen in diesem Bereich zu verringern. Als nächster Schritt folgt dann wieder der Schritt I. Bei gleichmäßigem Versatz der Nockensegmente der Welle und somit auch der Lamellen zueinander, vorausgesetzt, die einzelnen Lamellen weisen gleiche Abmessungen auf, wird somit eine sinusförmige Klemmbewegung der Lamellen erzeugt, wodurch die Fluidströmung durch den Schlauch erzeugt werden kann.

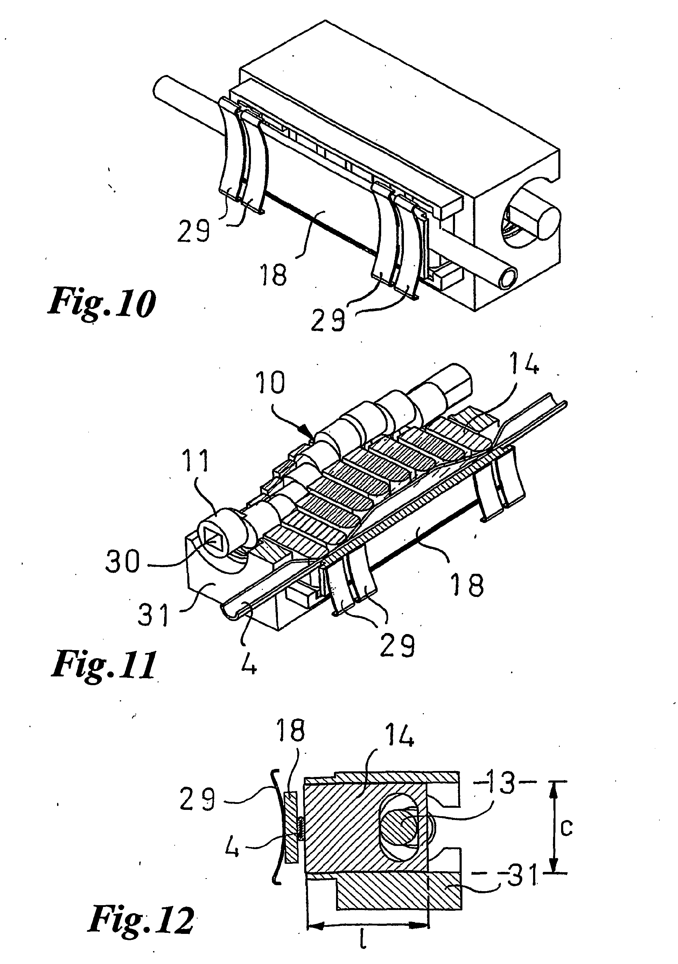

Die Figuren 10 bis 12 zeigen eine weitere Ausführungsform eines Teils einer Pumpvorrichtung. Bei dieser sind anstelle nur einer längs parallel zum Schlauch verlaufenden Blattfeder wie in Figur 6 gezeigt, endseitig quer zu diesem angeordnete Blattfederpaare 29 vorgesehen, die auf die Gegendruckplatte 18 drücken. Wie zu den vorigen Figuren beschrieben, üben sie einen Gegendruck zu den sich bewegenden Lamellen 14 aus, wie besonders Figur 11 zu entnehmen ist. In dieser Figur weist die Welle 10 an dem einen Ende 11 eine Vierkant-Innenöffnung 30 auf, in die ein Sensor eingefügt werden kann. Dieser ist dadurch an der relevanten Stelle und zugleich platzsparend und geschützt angeordnet.

In Figur 12 ist eine Ausbildung der Lamellen 14 im Längsschnitt gezeigt. Hier wird die Führung dadurch verbessert, dass die Lamellen über ihre gesamte Länge in dem die Lamellen aufnehmenden Gehäuseteil 31 geführt werden. Die Führungslänge wird damit also maximiert und die Führung verbessert.

Die Figuren 13 bis 16 zeigen verschiedene Prinzipskizzen von Wellen, auf die von einer Querschnittsseite aus aufgesehen wird. In Figur 13 ist ein durchgehender Kernbereich 22 vorgesehen, Die Nockensegmente 13 der Welle 10 sind um einen Winkel α von jeweils 40° zueinander versetzt. Zwischen den einzelnen Nockensegmenten ist hierdurch eine verhältnismäßig große Kontaktfläche 32 zur Erhöhung der Stabilität der Welle vorgesehen. Eine solche Kontaktfläche 32 ist in den Figuren 13 bis 16 jeweils gestrichelt dargestellt.

In Figur 13 beträgt der Hub h 4 mm, also der halbe Hub h/2 2 mm. Die Nockensegmente weisen einen Durchmesser von 6 mm auf. Der Kernbereich ist so groß gewählt, dass der von den rotierenden Nockensegmenten umlaufene Außendurchmesser d 10 mm beträgt. Der Kernbereich weist in dieser Ausführungsform etwa einen Durchmesser von 2 mm auf, so dass sich ein Verhältnis von Nockensegmentdurchmesser zu Kernbereichsdurchmesser von 3:1 ergibt. Der Außendurchmesser d entspricht in etwa der Lamellenhöhe bzw. dem Innendurchmesser der Durchgangsöffnung 15 durch eine Lamelle. Das Verhältnis von Lamellenhöhe zu Hub wird hierbei also bei etwa 3:1 liegen.

Bei der Ausführungsform nach Figur 14 ist ein Kernbereich von etwa Null vorgesehen, also die Nockensegmente so nebeneinander angeordnet, dass sie einander im mittleren Bereich beim Rotieren nicht überlappen. Der Versatz beträgt wieder 40°. Der Nockensegmentdurchmesser nun 4 mm. Der Hub ist auf konstant 4 mm gehalten. Der Außendurchmesser d beträgt jedoch nur noch 8 mm. Das Verhältnis von Nockensegmentdurchmesser zum Durchmesser des Kernbereichs lässt sich nicht errechnen, da kein durchgehender Kernbereich vorgesehen ist. Das Verhältnis von Lamellenhöhe zu Hub beträgt etwa 2,5:1.

Betrachtet man nun die Ausführungsform nach Figur 15, ist ersichtlich, dass die Nockensegmente sich nicht nur nicht im mittleren Bereich, also im Bereich einer gedachten Mittellinie 21. überschneiden oder treffen, in der Projektion der Welle in die Ebene, sondern vielmehr dort eine Öffnung 33 ausbilden. Um diese Öffnung rotieren sie. Der Nockensegmentdurchmesser kann dabei noch weiter vermindert werden auf hier z.B. 3 mm. Der Hub wird wiederum auf 4 mm gewählt. Der Außendurchmesser d beträgt hier 7 mm. Der Versatz zwischen den einzelnen Nockensegmenten beträgt weiterhin 40°; es sind also neun Nockensegmente vorgesehen. Das Verhältnis von Lamellenhöhe zu Hub beträgt somit etwa 2,25:1.

Eine weitere Verringerung des Nockensegmentdurchmessers ist in der Figur 16 vorgenommen, wo dieser nur noch 2 mm beträgt. Der Hub liegt weiterhin bei 4 mm und der Versatz bei 40°. Der Außendurchmesser beträgt 6 mm. Das Verhältnis von Lamellenhöhe zu Hub beträgt somit etwa 2:1. Da die Öffnung 33, um die die Nockensegmente rotieren, etwa dem Nockensegmentdurchmesser entspricht, also etwa 2 mm beträgt, ist die Stabilität der Welle 10 nicht so groß wie beispielsweise bei der Ausführungsform nach Figur 13 mit durchgehendem Kernbereich oder auch Figur 14 oder 15. Um die Kontaktflächen 32 zwischen den Nockensegmenten zu vergrößern, kann die Anzahl der Nockensegmente vergrößert werden, also der Versatz verringert, wie aus Figur 16 einfach vorstellbar ist. Die Wahl eines etwas größeren Verhältnisses von Lamellenhöhe zu Hub, wie z.B. in Figur 13, stellt einen guten Kompromiss zwischen möglichst kleinen mechanischen Bauelementen und einer möglichst großen mechanischen Stabilität dar.

Neben den im Vorstehenden beschriebenen und in den Figuren abgebildeten Ausführungsformen können noch weitere gebildet werden, bei denen jeweils eine Zwangsführung der Lamellen vorgesehen ist, also ein System zum Zurückziehen der Lamellen von einer rhythmisch zu klemmenden Leitung und bei denen ein großer Hub im Vergleich zu der Lamellengröße erzielt werden kann.

### Bezugszeichenliste

- 1: Pumpvorrichtung
- 2: Gehäuse
- 3: peristaltische Antriebseinrichtung
- 4: Schlauch
- 5: Einlegebereich
- 6: Drucksensor
- 7: Durchflusssensor
- 8: Pfeil
- 9: Motor
- 10: Welle
- 11: erstes Ende
- 12: zweites Ende
- 13: Nockensegment
- 14: Lamelle
- 15: Durchgangsöffnung
- 16: Stirnfläche
- 17: Außenseite
- 18: Gegendruckplatte
- 19: Feder
- 20: Klappe
- 21: Mittellinie
- 22: Kernbereich
- 23: Tonnenfeder
- 24: Scharnier
- 25: Mulde
- 26: Blattfeder
- 27: seitlicher Steg
- 28: seitlicher Steg
- 29: Blattfederpaar
- 30: Vierkant-Innenöffnung
- 31: Gehäuseteil
- 32: Kontaktfläche
- 33: Öffnung
- h: Hub
- c: Lamellenbautiefe
- 1: Lamellenlänge
- α: Versatz
- d: Außendurchmesser

## Patentansprüche

1. Pumpvorrichtung (1) mit einer peristaltischen Antriebseinrichtung (3) zum Pumpen eines Mediums durch eine Leitung (4) mit zumindest einem kompressiblen Abschnitt, enthaltend eine einstückige Welle (10) mit versetzt zueinander angeordneten Nocken und aufgefügten Lamellen (14), wobei eine Zwangsführung für die Lamellen (14) vorgesehen ist,
**dadurch gekennzeichnet, dass**
die Nocken Nockensegmente (13) sind und die Welle (10) kemwellenlos ohne einen durchgehenden Kernbereich oder zur Stabilitätserhöhung mit einem dünnen durchgehenden Kernbereich mit einem Durchmesser von unter 3 mm mit zueinander versetzten, aneinander angrenzenden Nockensegmenten (13) ausgebildet ist.

2. Pumpvorrichtung (1) nach Anspruch 1,
**dadurch gekennzeichnet, dass**
eine Gegendruckplatte (18) zum Anlegen der Leitung, insbesondere eines Schlauchs (4) und Gegenhalten des von den Lamellen (14) auf die Leitung bzw. den Schlauch (4) ausgeübten Drucks vorgesehen ist.

3. Pumpvorrichtung (1) nach Anspruch 2,
**dadurch gekennzeichnet, dass**
die Gegendruckplatte (18) durch eine oder mehrere Federn (19, 23, 26) innerhalb eines Gehäuses der Pumpvorrichtung (1) abgefedert ist, insbesondere durch Tonnenfedern (23), Blattfedern (26) oder eine andere Federart.

4. Welle (10) für eine Pumpvorrichtung (1) mit einer peristaltischen Antriebseinrichtung (3) nach einem der vorstehenden Ansprüche, wobei die Welle einstückig geformt ist,
**dadurch gekennzeichnet, dass**
die Welle (10) kernwellenlos ohne einen durchgehenden Kernbereich oder zur Stabilitätserhöhung mit einem dünnen durchgehenden Kernbereich mit einem Durchmesser von unter 3 mm mit zueinander versetzten, aneinander angrenzenden Nockensegmenten (13) ausgebildet ist.

5. Welle (10) nach Anspruch 4,
**dadurch gekennzeichnet, dass**
eine ungerade oder gerade Anzahl von Nockensegmenten (13) vorgesehen ist.

6. Welle (10) nach Anspruch 4 oder 5,
**dadurch gekennzeichnet, dass**
die Nockensegmente (13) so zueinander versetzt sind, dass nur ein Nockensegment einen maximalen Abstand zu einer fiktiven Mittellinie (21) der Welle (10) aufweist.

7. Welle (10) nach Anspruch 6,
**dadurch gekennzeichnet, dass**
ein gleichmäßiger Versatz (α) der Nockensegmente (13) vorgesehen ist, insbesondere ein Versatz von 40° bei neun Nockensegmenten (13).

8. Welle (10) nach einem der Ansprüche 4 bis 7,
**dadurch gekennzeichnet, dass**
die Welle (10) aus einem Kunststoffmaterial, insbesondere einem Kohlefaser-Werkstoff, einem glasfaserverstärkten Polymer oder einem anderen stabilen und maßhaltigen Material besteht.

9. Verwendung der Pumpvorrichtung (1) nach einem der Ansprüche 1 bis 3 als Infusionspumpe, Transfusionspumpe, für die Dialyse oder als Schlauchpumpe für andere medizinische Zwecke.

10. Verfahren zum Pumpen eines Mediums durch eine Leitung (4) mit zumindest einem kompressiblen Abschnitt unter Verwendung einer Pumpvorrichtung (1) mit einer peristaltischen Antriebseinrichtung (3) enthaltend eine einstückige Welle (10), die kernwellenlos ohne einen durchgehenden Kernbereich oder zur Stabilitätserhöhung mit einem dünnen durchgehenden Kernbereich mit einem Durchmesser von unter 3 mm mit zueinander versetzten, aneinander angrenzenden Nockensegmenten (13) und aufgefügten Lamellen (14) ausgebildet ist, wobei eine Zwangsführung für die Lamellen (14) vorgesehen ist und die Nockensegmente so zueinander versetzt sind, dass nur ein Nockensegment einen maximalen Abstand zu einer fiktiven Mittellinie der Welle aufweist, wobei zum Erzeugen einer Fluidströmung in der Leitung die Lamellen die in die Pumpvorrichtung eingelegte Leitung fortschreitend einseitig zusammendrücken, jedoch nicht vollständig abklemmen.

11. Verfahren nach Anspruch 10,
**dadurch gekennzeichnet, dass**
die Leitung so geklemmt wird, dass ein Volumen beim ersten und letzten Nockensegment dicht eingeschlossen wird und die übrigen Lamellen zur Volumenverkleinerung dienen.

12. Verfahren nach Anspruch 11,
**dadurch gekennzeichnet, dass**
die erste und letzte Lamelle als Ventil geschaltet und die übrigen Lamellen so eingestellt werden, dass in jeder Position zumindest ein schmaler Spalt zwischen Wänden der durch die Lamellen beaufschlagten Leitung verbleibt.

13. Verfahren nach einem der Ansprüche 10 bis 12,
**dadurch gekennzeichnet, dass**
ein Pumpvorgang in zwei Richtungen, einer ersten und einer entgegengesetzten zweiten Richtung, erfolgt.

14. Verfahren nach einem der Ansprüche 10 bis 13,
**dadurch gekennzeichnet, dass**
bei gleichmäßige Versatz der Nockensegmente eine sinusförmige Klemmbewegung der Lamellen zum Erzeugen der Fluidströmung durch die Leitung (4) erzeugt wird.

## Claims

1. Pumping device (1) having a peristaltic drive device (3) for pumping a medium through a line (4) having at least one compressible portion, containing a one-piece shaft (10) having an arrangement of mutually offset cams and joined-on lamellae (14), wherein a restricted guidance is provided for the lamellae (14),
**characterized in that**
the cams are cam segments (13) and the shaft (10) is of a core-shaft-less design without a continuous core region or for increased stability is designed with a thin continuous core region having a diameter of below 3 mm with mutually offset, mutually adjacent cam segments (13).

2. Pumping device (1) according to claim 1,
**characterized in that**
a counterpressure plate (18) is provided for laying-on of the line, in particular of a flexible tube (4) and for holding against the pressure exerted by the lamellae (14) on the line and/or flexible tube (4).

3. Pumping device (1) according to claim 2,
**characterized in that**
the counterpressure plate (18) is spring-mounted inside a housing of the pumping device (1) by means of one or more springs (19, 23, 26), in particular by means of barrel springs (23), leaf springs (26) or some other type of spring.

4. Shaft (10) for a pumping device (1) having a peristaltic drive device (3) according to one of the preceding claims, wherein the shaft is formed in one piece, **characterized in that**
the shaft (10) is of a core-shaft-less design without a continuous core region or for increased stability is designed with a thin continuous core region having a diameter of below 3 mm with mutually offset, mutually adjacent cam segments (13).

5. Shaft (10) according to claim 4,
**characterized in that**
an odd or even number of cam segments (13) is provided.

6. Shaft (10) according to claim 4 or 5,
**characterized in that**
the cam segments (13) are mutually offset in such a way that only one cam segment has a maximum spacing from a hypothetical centre line (21) of the shaft (10).

7. Shaft (10) according to claim 6,
**characterized in that**
a uniform offset (α) of the cam segments (13) is provided, in particular an offset of 40° in the case of nine cam segments (13).

8. Shaft (10) according to one of claims 4 to 7,
**characterized in that**
the shaft (10) is made of a plastics material, in particular a carbon fibre material, a glass-fibre-reinforced polymer or some other stable and dimensionally true material.

9. Use of the pumping device (1) according to one of claims 1 to 3 as an infusion pump, transfusion pump, for dialysis or as a tube pump for other medicinal purposes.

10. Method of pumping a medium through a line (4) having at least one compressible portion using a pumping device (1) having a peristaltic drive device (3) containing a one-piece shaft (10), which is of a core-shaft-less design without a continuous core region or for increased stability is designed with a thin continuous core region having a diameter of below 3 mm with mutually offset, mutually adjacent cam segments (13) and joined-on lamellae (14), wherein a restricted guidance is provided for the lamellae (14) and the cam segments are mutually offset in such a way that only one cam segment has a maximum spacing from a fictitious centre line of the shaft, wherein for generating a fluid flow in the line the line inserted into the pumping device is progressively compressed at one side, but not totally occluded, by the lamellae.

11. Method according to claim 10,
**characterized in that**
the line is squeezed in such a way that in the case of the first and last cam segment a volume is tightly enclosed and the remaining lamellae are used for the volume reduction.

12. Method according to claim 11,
**characterized in that**
the first and last lamella are operated as a valve and the remaining lamellae are set in such a way that in each position at least a narrow gap is left between walls of the line acted upon by the lamellae.

13. Method according to one of claims 10 to 12, **characterized in that**
a pumping action is effected in two directions, a first and an opposite second direction.

14. Method according to one of claims 10 to 13,
**characterized in that**
given a uniform offset of the cam segments a sinusoidal squeezing movement of the lamellae is produced for generating the fluid flow through the line (4).

## Revendications

1. Dispositif formant pompe (1) avec un système d'entraînement péristaltique (3) pour pomper un fluide à travers une conduite (4) présentant au moins une partie compressible, comportant un arbre d'un seul tenant (10) pourvu de cames décalées les unes par rapport aux autres et de lamelles rapportées (14), sachant qu'il est prévu un guidage forcé pour les lamelles (14),
**caractérisé en ce que** les cames sont des segments de cames (13) et l'arbre (10) est conçu sans âme, c'est-à-dire sans zone centrale continue ou, afin d'améliorer la stabilité, avec une mince zone centrale continue d'un diamètre inférieur à 3 mm, avec des segments de cames (13) décalés les uns par rapport aux autres mutuellement adjacents.

2. Dispositif formant pompe (1) selon la revendication 1, **caractérisé en ce qu'**il est prévu un plateau de contrepression (18) pour appliquer la conduite, en particulier un tuyau souple (4), et pour supporter la pression exercée par les lamelles (14) sur la conduite ou encore le tuyau souple (4).

3. Dispositif formant pompe (1) selon la revendication 2, **caractérisé en ce que** le plateau de contrepression (18) est suspendu par un ou plusieurs ressorts (19, 23, 26) à l'intérieur d'un boîtier du dispositif formant pompe (1), notamment par des ressorts bombés (23), des ressorts à lames (26) ou un autre type de ressorts.

4. Arbre (10) pour un dispositif formant pompe (1) avec un système d'entraînement péristaltique (3) selon l'une des revendications précédentes, sachant que l'arbre est formé d'un seul tenant,
**caractérisé en ce que** l'arbre (10) est conçu sans âme, c'est-à-dire sans zone centrale continue ou, afin d'améliorer la stabilité, avec une mince zone centrale continue d'un diamètre inférieur à 3 mm, avec des segments de cames (13) décalés les uns par rapport aux autres mutuellement adjacents.

5. Arbre (10) selon la revendication 4, **caractérisé en ce qu'**il est prévu un nombre impair ou pair de segments de cames (13).

6. Arbre (10) selon la revendication 4 ou 5, **caractérisé en ce que** les segments de cames (13) sont décalés les uns par rapport aux autres de telle sorte qu'un seul segment de came présente une distance maximale par rapport à un axe médian fictif (21) de l'arbre (10).

7. Arbre (10) selon la revendication 6, **caractérisé en ce qu'**il est prévu un décalage uniforme (α) des segments de cames (13), notamment un décalage de 40° pour neuf segments de cames (13).

8. Arbre (10) selon l'une des revendications 4 à 7, **caractérisé en ce que** l'arbre (10) est constitué d'une manière plastique, notamment d'un matériau à base de fibres de carbone, d'un polymère renforcé par des fibres de verre ou d'un autre matériau mécaniquement et dimensionnellement stable.

9. Utilisation du dispositif formant pompe (1) selon l'une des revendications 1 à 3 comme pompe de perfusion, comme pompe de transfusion, pour la dialyse ou comme pompe péristaltique à d'autres fins médicales.

10. Procédé pour pomper un fluide à travers une conduite (4) présentant au moins une partie compressible, en utilisant un dispositif formant pompe (1) avec un système d'entraînement péristaltique (3) comportant un arbre d'un seul tenant (10) qui est conçu sans âme, c'est-à-dire sans zone centrale continue ou, afin d'améliorer la stabilité, avec une mince zone centrale continue d'un diamètre inférieur à 3 mm, avec des segments de cames (13) décalés les uns par rapport aux autres mutuellement adjacents et des lamelles rapportées (14), sachant qu'il est prévu un guidage forcé pour les lamelles (14) et que les segments de cames sont décalés les uns par rapport aux autres de telle sorte qu'un seul segment de came présente une distance maximale par rapport à un axe médian fictif de l'arbre, sachant qu'afin de produire un écoulement de fluide dans la conduite, les lamelles compriment progressivement d'un côté la conduite insérée dans le dispositif formant pompe, mais ne la ferment pas totalement par serrage.

11. Procédé selon la revendication 10, **caractérisé en ce que** la conduite est serrée de telle sorte qu'un volume est enfermé en étanchéité au niveau du premier et du dernier segment de came et que les autres lamelles servent à réduire le volume.

12. Procédé selon la revendication 11, **caractérisé en ce que** la première et la dernière lamelle sont montées comme soupape et les autres lamelles sont réglées de telle sorte que, dans chaque position, il reste au moins un passage étroit entre les parois de la conduite sollicitée par les lamelles.

13. Procédé selon l'une des revendications 10 à 12, **caractérisé en ce qu'**on effectue un processus de pompage dans deux directions, une première direction et une deuxième direction opposée.

14. Procédé selon l'une des revendications 10 à 13, **caractérisé en ce que,** avec un décalage uniforme des segments de cames, on produit un mouvement de serrage sinusoïdal des lamelles afin de produire l'écoulement de fluide à travers la conduite (4).
